# EUROPEAN PATENT APPLICATION

(11) **EP 2 000 132 A1**
(43) Date of publication of application: **10.12.2008**
(21) Application number: 07011020.0
(22) Date of filing: 05.06.2007
(51) Int. Cl.: A61K 9/28

(54) **Solid pharmaceutical formulation**

(71) Applicant: The Jordanian Pharmaceutical Manufacturing Co., 11710 Naor (JO)
(72) Inventor: Badwan, Adnan, Dr., 11185 Amman (JO); Al-Remawi, Mayyas, Dr., 13713 Russiefa (JO)
(74) Representative: Winkler, Andreas Fritz Ernst

(57) **Abstract**

The present invention relates to a solid pharmaceutical formulation comprising at least one hydrophilic water-soluble superdisintegrating oligo-polymer having a molecular weight in the range of 200-3000 Da and having from 3 to 20 monomeric repeating units forming the oligo-polymer as matrix and at least one active agent dispersed throughout the matrix, wherein the oligo-polymer simultaneously dissolves and disintegrates in a liquid.

## Description

The present invention relates to a solid pharmaceutical formulation which can be orally administered. Solid formulations which can be orally administered are well known in the prior art, for example tablets. For specific applications, it is preferred or necessary that the orally administered formulation can be rapidly dissolved in the oral cavity. This is for example true for those active agents contained in the formulation suffering from first pass effects. Some materials exhibit this first pass effect which is a high metabolism effect occurring either in the small intestine or in the liver prior to the availability of the drug in the blood stream. One of the methods used to get rid of this effect is to make the drug absorbed from the buccal route, i.e. in the oral cavity. Sometimes also a rapid onset of drug action is required. Further, rapid dissolution is of particular importance for infants, children and elderly people who face problems in swallowing solid dosage forms. Administration of a solid formulation does not require the injection of a cup of water to swallow, i.e. it is more convenient for most patients.

Known techniques for rapid dissolving systems include lyophilization, solid-state dissolution and effervescent tablets. These techniques are slow, expensive and add additional costs to the tablet. Effervescent dosage forms tend to bubble in the mouth leaving an uncomfortable feeling.

It is an object of the present invention to provide a solid pharmaceutical formulation which overcomes the drawbacks of the prior art. Especially a solid pharmaceutical formulation sha>ll be provided which rapidly dissolves and disintegrates in the oral cavity.

This object is achieved by a solid pharmaceutical formulation comprising at least one hydrophilic water-soluble superdisintegrating oligo-polymer having a molecular weight in the range of 200-3000 Da and having from 3 to 20 monomeric repeating units forming the oligo-polymer as matrix and at least one active agent dispersed throughout the matrix, wherein the oligo-polymer simultaneously dissolves and disintegrates in a liquid.

Most preferably the formulation is a tablet.

In one embodiment the density of the formulation is 0.1 to 0.5 g/ml.

Preferably, the oligo-polymer is selected from the group consisting of saccharide, chitin, starch, alginic acid, crospovidon, sodium starch glycolate, hydroxypropyl cellulose, croscar-melose sodium, carboxymethyl cellulose calcium, cellulose, guar gum, and derivatives thereof.

In one preferred embodiment further additives are added selected from the group consisting of effervescent agents, water absorbents, surfactants, wetting agents, binding agents, and flavoring agents. Preferred additives added to the inventive formulation may be selected from effervescent agents, such as sodium bicarbonate, tartaric acid, or water absorbent, such as silicon dioxide, or surfactants, such as sodium lauryl sulfate.

It is further proposed that the formulation is coated, preferably with a polymeric coat.

Additionally, it is most preferred that the liquid is water or a body fluid.

Inventive is further the use of the solid pharmaceutical formulation for oral administration to a subj ect.

Surprisingly it was found that according to the present invention a solid pharmaceutical formulation may be provided, preferably a tablet, which disintegrates and dissolves in a manner of just a few seconds once placed in the oral cavity. The rapid dissolving formulation provides prompt drug release within a few seconds in the mouth cavity. The inventive formulation depends strongly on inherent properties of the hydrophilic oligo-polymers. These oligo-polymers have the ability to swell and disintegrate very rapidly. The low molecular weight thereof also facilitates the dissolution after disintegration in a rapid period of time.

It is an essential feature of the present invention that the oligo-polymer used has the property of substantially simultaneous dissolution and disintegration that enables rapid disintegration and rapid dissolution at the same time. Thus, such an excipient can be used in rapid oral dissolving systems.

Additionally, the solid pharmaceutical formulation has many of the advantages of a regular solid pharmaceutical formulation, such as regular oral tablet, including convenient size, stability, dispensability, transportability and the capability to easily alter the dose. Further, the formulation can be easily administered.

Surprisingly it was found that the release of the active agent from the solid pharmaceutical formulation after oral administration occurred spontaneously upon placing this dosage form in the mouth, where the saliva will serve to rapidly dissolve the formulation. As the rapid dissolution of the formulation avoids swallowing of solid dosage forms, the inventive formulation is especially preferred for infants, children and elderly people who face problems in swallowing solid dosage forms.

Generally, it is assumed that the inventive formulation is based on two important opposing factors, i.e. swellability and solubility.

Many known hydrophilic polymers have the tendency to swell very rapidly in the particulate level independently. These particles are not soluble but swellable to the level that permits the tablet to disintegrate. This tendency is found in most disintegrants. In fact, the known disintegrants possess large molecular weights. The high molecular weight of these polymers (for example chitin > 500.000, crospovidone > 1.000.000, sodium starch glycolate > 500.000) results in an increase in disintegration power, but the hydrophilic polymer solubility is decreased. Monomer structures related to the hydrophilic polymers, e.g. acetyl glucosamine for chitin and glucose for cellulose have a high solubility. This increase in solubility results in a tremendous decrease in swellability. Thus, monomers posses no disintegration power compared to their polymers, while polymers have no solubility power compared to monomers.

Hydrophilic oligo-polymers which are to be used in inventive formulations are polymers having a low molecular weight. Surprisingly, it has been found that there is a optimum molecular weight where the polymer has disintegration power and solubility that permits the formation of a rapidly dissolving matrix. Where the oligo-polymer swells in a rate which is higher than the dissolution rate. The particle swelling leads to matrix formulation disintegration in a rapid manner. After the fine particles are dispersed in the mouth they tend to dissolve rapidly. Then, a rapid dissolving system is formed using low molecular weights of the known superdisintegrants.

For inventive solid pharmaceutical formulation it has been found that, where the formulation is introduced into an aqueous environment, the matrix is substantially completely disintegrated and dissolved within less than about 2-40 seconds.

The oligo-polymers utilized in the present invention can be produced either by partial depolymerization of the high molecular weight polymers or polymerization of the monomer structures to the optimum degree of polymerization. The optimum degree of polymerization of course varies and depends on the chemical structure of the polymer. The oligo-polymers may be further reprocessed by different techniques known in the art to increase the disintegration and rapid dissolving power, such as freeze drying, spray drying, etc.

Additional advantages and features of the subject-matter of the present invention will become apparent from the following detailed description on the basis of examples with reference to the accompanying drawing, wherein
Figure 1 is an infrared spectrum of chitin crude (reference) mentioned in example 1;
Figure 2 is an infrared spectrum of oligochitin prepared in example 1 using method 1;
Figure 3 is an infrared spectrum of oligochitosan used in example 1 to prepare oligochitin used in method 1; and
Figure 4 is a graph illustrating the dependency of dissolution and disintegration on molecular weight of a hydrophilic polymer.

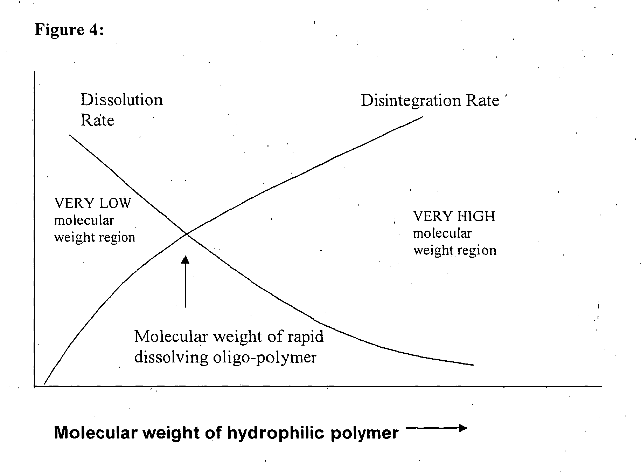

In figure 4 linear polyglucose (i.e. cellulose) as an example of a hydrophilic polymer and its monomeric glucose where chosen.

Glucose is a monosaccharide and it is the main building block of cellulose. Thus, it has the lowest molecular weight in a poly (glucose) polymer. Glucose has a high water solubility and when compressed into a form of compact it will have a high binding power and results in a hard solid mass. When exposed to water it will dissolve slowly but it will never disintegrate, i.e. it will be located in the left side of the graph of figure 4. Cellulose is a high molecular weight polyglucose polymer. It is a straight chain polymer consisting of glucose units linked with a beta-glycosidic bond. Cellulose is insoluble in water although its hydrophilic but it swells in water. Once cellulose powder is compressed in a compact and exposed to water, cellulose powder will swell and disintegrate but it will not dissolve. Due to its high molecular weight, cellulose will be located in the right side of the graph of figure 4.

The dissolution is inversely related with the molecular weight and disintegration is directly related with the molecular weight. Then an intersection point or range of a suitable molecular weight can be located where dissolution equals disintegration at a specific range of hydrophilic polymer molecular weight. It was found that for typical hydrophilic oligo-polymers the range where dissolution and disintegration takes place more or less simultaneously is between 200-3000 Da.

### Examples

The following examples further illustrate solid pharmaceutical formulations according to the present invention, however these examples are not to be construed as limitation of this invention and have been prepared without active agent. Oligo-chitin was taken as a model oligo-polymer.

### Example 1

### Preparation method of chitin oligosaccharide (method 1)

50 gram of chitosan oligosaccharide HCl having an average molecular weight of 2300 dalton was dissolved in 100 ml water and titrated with 1 M NaOH solution to adjust the pH to 8-9. Then, ethanol was added in excess to precipitate chitosan base. After suction filtration, the filtrate was added to acetic anhydride and was left for a few minutes under stirring. The precipitated substance was filtrated and excess acetic acid was rinsed using acetone. The sample was dried in an oven at 50°C for 30 minutes.

### Preparation method of chitin oligo saccharide (method 2)

50 gram of chitin having a high molecular weight in the range of more than 1.000.000, obtained as crude polymer, was placed in 100 ml concentrated HCl and heated for three hours, until the material dissolved completely. The pH was adjusted to 8-9 using 6 M NaOH solution. The material was extracted by the addition of acetone. Two layers were formed. The aqueous layer was freeze-dried for one day. The dry powder was collected as chitin oligosaccharide.

### Example 2

### Characterization of chitin oligo saccharide

Three samples were evaluated using FTIR techniques; the prepared chitin in example 1 using method 1 (product), chitosan oligosaccharide (reactant material) and chitin with high molecular weight (reference). Infrared spectra were obtained using FTIR 480, Jasko, Japan. Fourier transform infrared spectrometer was utilized under ambient conditions at room temperature and using KBr disc: Samples were placed in an oven at 105°C for three hours before carrying out any measurements to get rid of moisture. Approximately 500 mg of KBr and 5 mg of sample powder were blended with pestle and mortar for about three minutes. The sample disc was prepared at a pressure of 10 tons for about 1 minute.

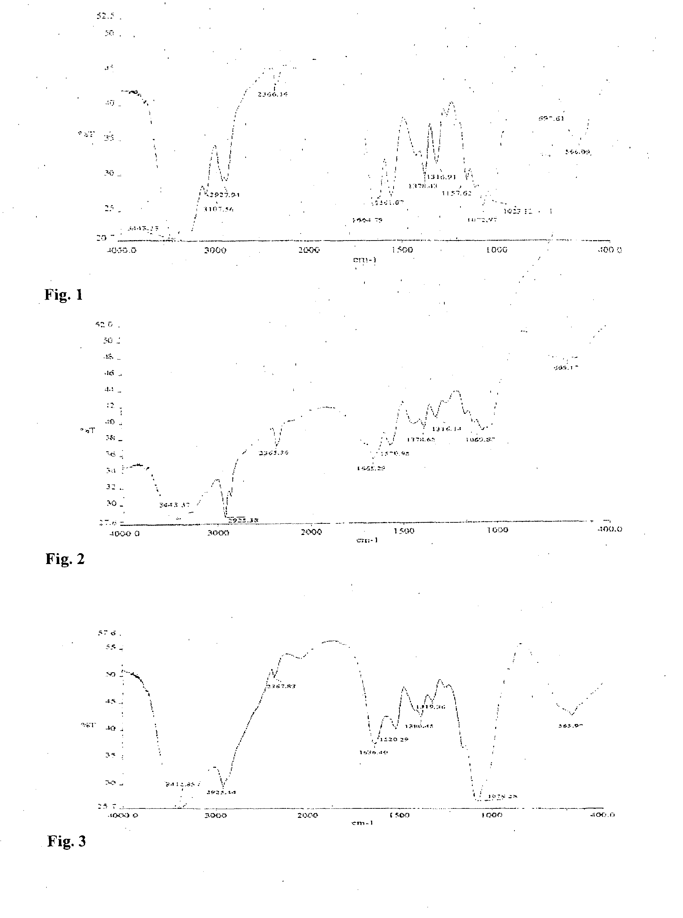

Figures 1 to 3 indicate the conversion of chitosan oligosaccharide to chitin oligosaccharide.

### Example 3

### Rapid dissolving matrix testing

Chitin prepared in example 1, method 1 and 2, was compacted using a Manesty single punch compression machine, Great Britain. The tablets produced were circular with diameter of 13 mm. The compression force was 35 kN.

The simple method for testing was adopted for the purpose of the proof of concept. The tablet was placed in a beaker containing about 500 ml water. Then the water was gently stirred using a spatula to facilitate the dispersion and the solution for one minute. For the chitin oligosaccharide prepared according to method 1 it was found that the tablet completely disintegratd and dissolved already after 30 seconds, wherein for the tablet produced according to method 2 dissolution and disintegration were completed after already 40 seconds.

Thus, it is demonstrated that the inventive solid pharmaceutical formulation can be rapidly dissolved and disintegrated after oral administration to result in the advantages outlined above.

The features disclosed in the foregoing description, in the claims and/or in the accompanying drawings may, both separately and in any combination thereof, be material for realizing the invention in diverse forms thereof.

## Claims

1. Solid pharmaceutical formulation comprising at least one hydrophilic water-soluble superdisintegrating oligo-polymer having a molecular weight in the range of 200-3000 Da and having from 3 to 20 monomeric repeating units forming the oligo-polymer as matrix and at least one active agent dispersed throughout the matrix, wherein the oligo-polymer simultaneously dissolves and disintegrates in a liquid.

2. Solid pharmaceutical formulation according to claim 1, wherein the formulation is a tablet.

3. Solid pharmaceutical formulation according to claim 1 or 2, wherein the density of the formulation is 0.1 to 0.5 g/ml.

4. Solid pharmaceutical formulation according to any of the preceding claims, wherein the oligo-polymer is selected from the group consisting of saccharide, chitin, starch, alginic acid, crospovidon, sodium starch glycolate, hydroxypropyl cellulose, croscar-mellose sodium, carboxymethyl cellulose calcium, cellulose, guar gum, and derivatives thereof.

5. Solid pharmaceutical formulation according to any of the preceding claims, wherein further additives are added selected from the group consisting of effervescent agents, water absorbents, surfactants, wetting agents, binding agents, and flavoring agents.

6. Solid pharmaceutical formulation according to any of the preceding claims, wherein the formulation is coated, preferably with a polymeric coat.

7. Solid pharmaceutical formulation according to any of the preceding claims, wherein the liquid is water or a body fluid.

8. Use of a solid pharmaceutical formulation according to any of the preceding claims for oral administration to a subject.
